# EUROPEAN PATENT APPLICATION

(11) **EP 2 829 277 A1**
(43) Date of publication of application: **28.01.2015**
(21) Application number: 13178151.0
(22) Date of filing: 26.07.2013
(51) Int. Cl.: A61K 36/77, A61K 45/06, A61K 31/353, A61K 36/00, A61K 36/185, A61K 36/45, A61K 36/54, A61K 36/736

(54) **Use of a-type proanthocyanidins in treating a mineralocorticoid receptor related disease**

(71) Applicant: Natac Biotech, S.L., 28049 Madrid (ES)
(72) Inventor: Martín Fernández, Beatriz, E-28040 Madrid (ES); De las Heras Jiménez, Natalia, E-28040 Madrid (ES); Pinilla Rosas, José María, E-28049 Madrid (ES); De la Fuente García, Esther, E-28049 Madrid (ES); Quintela Fernández, José Carlos, E-28049 Madrid (ES); Lahera Juliá, Vicente, E-28040 Madrid (ES)
(74) Representative: ABG Patentes, S.L.

(57) **Abstract**

The invention refers to the use of an A-type proanthocyanidin to prevent and/or treat a mineralocorticoid receptor related disease or disorder.

## Description

### FIELD OF THE INVENTION

The invention concerns a new use of A-type proanthocyanidins in medicine, particularly in the treatment and/or prevention of mineralocorticoid receptor related diseases. Therefore, the invention fits into chemical pharmaceutical field.

### BACKGROUND

Mineralocorticoid receptor (MR) is an intracellular receptor activated by mineralocorticoids such as aldosterone and deoxicorticosterone, as well as glucocorticoids such as cortisol and cortisone. This receptor can be activated by progestin too.

Once the ligand, aldosterone, is bonded to mineralocorticoid receptor, translocation to the cellular nucleus, homodimerization, and binding to hormonal response elements occurs which activate target genes involved in different physiological actions such as increasing of Na⁺ (together with water) reabsorption in the distal convoluted tubule. Serum and glucocorticoid regulated kinase (SGK1) is the most firmly established mediator of aldosterone action. It has been shown that aldosterone induces SGK-1 gene expression, which triggers a cascade that leads to the absorption of sodium and water through the sodium channel in the apical membrane of tubular epithelial cells.

Primary and secondary hyperaldosteronism are conditions in which the adrenal gland releases an excess of the aldosterone hormone.

Mineralocorticoid receptors have also been localized in a number of nonepithelial tissues, particularly in the cardiovascular system and central nervous system. While the properties of the MRs in these tissues are largely similar, the effects they mediate are extremely diverse. In contrast to its established effects on electrolyte balance in epithelial tissue, aldosterone in the cardiovascular system promotes cardiac hypertrophy, fibrosis and abnormal vascular endothelial function. In the central nervous system it appears to regulate blood pressure, salt appetite and sympathetic tone.

Spironolactone and eplerenone are antagonists of mineralocorticoid receptor. Their binding to the receptor changes the structural conformation avoiding the transcriptional response. Nowadays, these medicines are used in the standard therapy for cardiac hypertrophy and heart failure but many adverse effects are found, for example stomach bleeding, gynecomastia, menstrual irregularities, testicular atrophy, ataxia and erectile dysfunction. Both spironolactone and eplerenone chemical structures are based in an steroid ring, as mineralocoriticoids, glucocorticoids and estrogens. These structural characteristics are probably related with their antagonism of the mineralocorticoid receptor and partial estrogen receptor agonism. This latter effect is the cause for some of their side effects. Therefore, new antagonists of mineralocorticoid receptor are needed.

Polyphenols are abundant micronutrients in our diet and there are evidences for their role in the prevention of various cardiovascular diseases such as hypertension, dyslipidemia and secondary alterations diabetes, obesity and/or insulin resistance. Polyphenols are widely spread in the vegetable kingdom. Several studies have been conducted to measure the cardiovascular beneficial effects of polyphenols in compounds of our diet such as chocolate, wine, tea and olive oil derivatives. In addition, polyphenols have several other specific biological actions that are yet poorly understood (anti-inflammatory, platelet anti-aggregate and antithrombotic) but have shown its benefit against atherosclerotic development and alterations related to cardiovascular risk factors.

Proanthocyanidins (PACS), also known as condensed tannins, are part of the chemical family of polyphenols. Proanthocyanidins are widely distributed in nature, being present in fruits, berries, vegetables, legume seeds, cereal grains, spices, and some beverages such us wine or tea.

From a structural point of view, Proanthocyanidins are oligomers or polymers composed by flavan-3-ol monomer subunits linked together. According to their composition they can be subdivided into three different families: Procyanidins - composed exclusively of catechin and epicatechin monomers-, prodelphinidins - containing at least one unit of gallocatechin or epigallocatechin together with units of catechin or epicatechin- and properlagonidins - containing at least one unit of afzelechin or epiafzelechin together with units of catechin or epicatechin.

According to the interflavonoid linkages between the different monomers, Proanthocyanidins can be subdivided into two main groups: Type A and type B. As shown in the following figure, B-type Proanthocyanidins present single bonds or linkages between carbon C-4 of one subunit (the upper subunit in the following figure) and carbon C-6 or C-8 from the other unit (lower subunit in the following figure) [i.e. 4→6 or 4→8 linkage]. A-type-Proanthocyanidins possess an additional bond through oxygen atoms between C-2 of the one subunit (the upper subunit in the following figure) and C-5 or C-7 of the other unit (lower subunit in the following figure) [i.e. 2→O→5 or 2→O→7 linkage]. A-type proanthocyanidinds can then be defined as a Proanthocyanidin in which at least two flavan-3-ol subunits are linked together by two interflavanoid bonds.

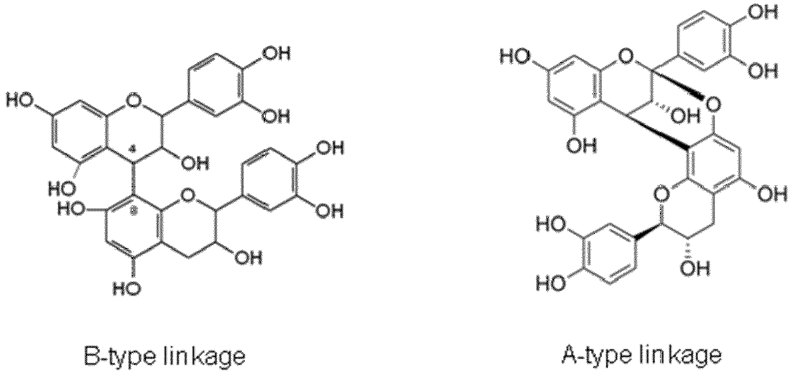

The above figure, provided to illustrate both types of linkages, shows the chemical structures of a B-type proanthocyanidin dimer (4→8) and an A-type proanthocyanidin dimer (4→8, 2→O→7).

Whereas B-type Proanthocyanidins are widely spread in different plants, A-type PACS are limited to a few botanical species.

Many studies relate generally to the beneficial effects of proanthocyanidins-rich extracts. For instance, it has been reported the use of proanthocyanidins in the treatment of occlusive thrombosis (WO 2007/002928A2), obstructive lung disease, asthma and allergic rhinitis (WO 2012/014165A1), ischemia and reperfusion (WO 2009/128738). The use of phospholipid complexes of oligomeric proanthocyanidins in heart and brain infarct has been described in patent application WO 99/29331A1. Patent application EP 2165712A1 has described an almond skin extract with antioxidant and anti-glycemic properties. Particularly, the use of A-type proanthocyanidins to prevent cellular ageing has been described in the patent application JP2003252745A and their use for healing wounds and injuries of various origins is disclosed in the patent application EP 0210785A1. Patent EP1713467B1 describes the use of A-type proanthocyanidins for treating or preventing of nitric oxide related pathologies. It has been also described the use of proanthocyanidins together with Centella asiatica in the treatment and prevention of atherosclerotic plaques especially in arteries (EP2464348A1) and together with certain vitamins and soy isoflavones improve cardiovascular alterations (WO 2006/032115A2).

### BRIEF DESCRIPTION OF THE INVENTION

The inventors of the present invention have surprisingly found and demonstrated that A-type proanthocyanidins are antagonists of mineralocorticoid receptor.

Therefore, one aspect of the present invention relates to A-type proanthocyanidins for use in treating and/or preventing a mineralocorticoid receptor related disease or disorder.

Another aspect of this invention refers to a pharmaceutical composition comprising at least one A-type proanthocyanidin and at least one pharmaceutically acceptable excipient for use in treating and/or preventing a mineralocorticoid receptor related disease or disorder.

Another aspect of this invention refers to the use an A-type proanthocyanidin for the manufacture of a medicament for the treatment and/or prevention of a mineralocorticoid receptor related disease or disorder.

Additional aspects of the invention refer to an isolated A-type proanthocyanidin or a natural extract comprising at least 5% in weight of proanthocyanidins, wherein at least 2% of the linkages of said proanthocyanidins are A-type linkages, in the above-mentioned uses and methods.

These aspects and preferred embodiments thereof are additionally also defined hereinafter in the detailed description, as well as in the claims.

### BRIEF DESCRIPTION OF THE FIGURES

Fig. 1: represents mineralocorticoid receptor expression in vehicle (V), aldosterone 1nm (A1nm), spironolactone 100nm (S100nm), aldosterone + spironolactone (A1nm+S100nm), A-type proanthocyanidins (PA2), B-type proanthocyanidins (PB2) and catechins (C90). Concentrations: 10nm (P10nm), 100nm (P100nm) and 1µM (P1µM) alone or together with aldosterone 1 nm.
Fig. 2: HW/BW; heart weight/100 g body weight in vehicle (CONTROL), aldosterone (ALDO), aldosterone + apple extract (AL+AP), and aldosterone + cinnamon extract (AL+CN), (*p<0,05 vs control, #p<0,05 vs aldosterone).
Fig. 3: LVEDP, left ventricular end-diastolic pressure in vehicle (CONTROL), aldosterone (ALDO), aldosterone + apple extract (AL+AP) and aldosterone + cinnamon extract (AL+CN), (*p<0,05 vs control, #p<0,05 vs aldosterone, +p<0,05 vs aldosterone + apple).
Fig. 4: HW/BW; heart weight/100 g body weight in vehicle (CONTROL), aldosterone (ALDO), and aldosterone + cranberry extract (AL+CR), (*p<0,05 vs control, #p<0,05 vs aldosterone).
Fig. 5: LVEDP, left ventricular end-diastolic pressure in vehicle (CONTROL), aldosterone (ALDO), and aldosterone + cranberry extract (AL+CR), (*p<0,05 vs control, #p<0,05 vs aldosterone).
Fig. 6: SGK-1 mRNA levels in vehicle (CONTROL), aldosterone (ALDO), and aldosterone + cranberry extract (AL+CR), (*p<0,05 vs control, #p<0,05 vs aldosterone).

### DETAILED DESCRIPTION OF THE INVENTION

In the context of the present invention, the following terms have the meaning detailed below.

As used herein, the terms "treat", "treating" and "treatment" include the eradication, removal, reversion, alleviation, modification, or control of a mineralocorticoid receptor related disease or disorder, after its onset.

As used herein, the terms "prevention", "preventing", "preventive" "prevent" and "prophylaxis" refer to the capacity of a therapeutic to avoid, minimize or difficult the onset or development of a mineralocorticoid receptor related disease or disorder before its onset.

Therefore, by "treating" or "treatment" and/or "preventing" or "prevention", as a whole, is meant at least a suppression or an amelioration of the symptoms associated with the condition afflicting the subject, where suppression and amelioration are used in a broad sense to refer to at least a reduction in the magnitude of a parameter, e.g., symptom associated with the condition being treated, i.e. a mineralocorticoid receptor related disease. As such, the method of the present invention also includes situations where the condition is completely inhibited, e.g., prevented from happening, or stopped, e.g., terminated, such that the subject no longer experiences the condition.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The "mineralocorticoid receptor related disease or disorder" is a disease or disorder mediated by the mineralocorticoid receptor (MR). The MR is expressed in many tissues, such as the kidney, colon, heart, central nervous system (hippocampus), brown adipose tissue and sweat glands. Therefore the mineralocorticoid receptor related disease or disorder may be related with the kidney, colon, heart, central nervous system (hippocampus), brown adipose tissue or sweat glands.

Representative examples of mineralocorticoid receptor related diseases or disorders include, but are not limited to, cardiac hypertrophy, cardiac fibrosis, left ventricle diastolic dysfunction, heart failure, heart attack, acute coronary syndromes, hypertension (such as pulmonary hypertension), arteriosclerosis, aterotrombosis, ateromatosis, diseases or disorders related to steroids hormones, chronic kidney failure, acute kidney failure and nephritic syndrome and its hepatosplacnic gastro-intestinal alterations.

In a preferred embodiment, the mineralocorticoid receptor related diseases or disorders are selected from the group consisting of cardiac hypertrophy, cardiac fibrosis, left ventricle diastolic dysfunction, heart failure, heart attack and acute coronary syndromes.

In another preferred embodiment, the mineralocorticoid receptor related disease or disorder is hypertension, including pulmonary hypertension.

In another preferred embodiment, the mineralocorticoid receptor related diseases or disorders are selected from the group consisting of arteriosclerosis, aterotrombosis and ateromatosis.

The "mineralocorticoid receptor" has affinity for the steroid hormones, particularly for the mineralocorticoids and the glucocorticoids.

Therefore, the mineralocorticoid receptor related diseases or disorders refer in another embodiment of the invention to diseases or disorders related to the mineralocorticoids (such as aldosterone, progesterone and deoxycorticosterone) and the glucocorticoids (such as cortisol and cortisone). Diseases or disorders related to the mineralocorticoids and the glucocorticoids are preferably selected from primary and secondary hyperaldosteronism, non-aldosterone mediated hypermineralocorticoidism, hypercortisolsm and hyperglucocorticoism.

Particularly preferred are diseases or disorders related with the aldosterone mineralocorticoid which are selected from primary and secondary hyperaldosteronism.

The term "A-type proanthocyanidins" refers to an oligomer composed of monomeric subunits of flavan-3-ol of formula: 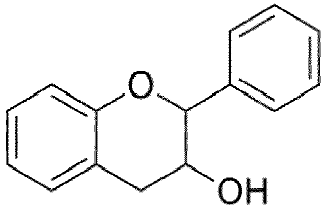 wherein at least two of the monomeric subunits are linked by an A-type interflavan linkage (4→6; 2→O→5), (4→6; 2→O→7), (4→8; 2→O→5), or (4→8; 2→O→7).

Therefore, the term "A-type proanthocyanidins" refers to procyanidins, prodelphinidins, and/or properlagonidins wherein at least two of their flavan-3-ol subunits are linked together by two interflavanoid bonds. The flavan-3-ol subunits can be selected from catechin, epicatechin, gallocatechin, epigallocatechin, afzelechin and epiafzelechin.

The A-type proanthocyanidin may be isolated. The term "isolated A-type proanthocyanidin" means that said A-type proanthocyanidin is substantially separated or purified from other compounds present in a natural composition in which the A-type proanthocyanidins naturally occur. The term "isolated A-type proanthocyanidin" thus encompasses A-type proanthocyanidin or proanthocyanidins extracted by standard techniques of extraction and/or purified by standard purification methods. The term also embraces A-type proanthocyanidins prepared by chemical synthesis or semisynthesis. Purity levels for the "isolated A-type proanthocyanidin" are preferably above 50%, more preferably above 70%, most preferably above 90%.

An A-type proanthocyanidins-rich extract in the sense of the invention refers to a natural extract comprising at least 5% in weight of proanthocyanidins, wherein at least 2% of the linkages of said proanthocyanidins are A-type linkages.

In a preferred embodiment, the A-type proanthocyanidin is comprised in a natural extract. In a most preferred embodiment the natural extract of the invention comprises at least 5% in weight of proanthocyanidins according to the analytical method described in European Pharmacopeia 7.0, 01/2008:1220, wherein at least 2% of the linkages of said proanthocyanidins are A-type linkages. Preferably the analysis of the type of linkages is performed by the HPLC-based method described in Gu, L. et al., Journal of Agricultural and Food Chemistry, 2003, 51 (25), 7513-7521; and Gu, L. et al., Journal of Agricultural and Food Chemistry, 2002, 50 (17), 4852-4860.

In a preferred embodiment, the natural extract comprises at least 10% weight of proanthocyanidins. In a more preferred embodiment, the natural extract comprises at least 20% weight of proanthocyanidins, most preferably at least 25%, at least 30%, at least 35%, at least 40%, at least 45%, at least 50%, at least 55%, at least 60%, at least 65%, at least 70%, at least 80%, or at least 85% weight of proanthocyanidins. In a even most preferred embodiment, the percentage of proanthocyanidins is about 86% weight.

In other preferred embodiments, at least 3%, 4%, or 5% of the linkages of the proanthocyanidins present in the natural extract are A-type linkages. Most preferably, at least 10%, at least 15%, at least 20%, at least 25%, at least 30%,at least 35%, at least 40%, at least 45%, at least 50%, at least 55%, at least 60%, or at least 65% of the linkages of the proanthocyanidins present in the natural extract are A-type linkages. In a even most preferred embodiment, the percentage of A-type linkages is about 66%.

A "natural extract" refers herein to a solution or preparation containing the active principles of natural species such as a plant, a fruit or the like. Preferred natural extracts comprising A-type proanthocyanidins are selected from horse-chestnut *(Aesculus hippocastanum* L.) extract, cinnamon extract (Cinnamomum verum) extract, almond (*Prunus dulcis* (Mill.) D.A.Webb.) extract, avocado (*Persea americana* Mill.) extract, peanut (*Arachis hypogaea* L*.*) extract and cranberry (*Vaccinium macrocarpon* L.) extract. Cranberry extract is particularly preferred.

A "natural extract" in the sense of the invention also encompasses manipulated natural extracts, for instance by adding additional components, mixing different natural extracts, etc.

The method described in the European Pharmacopeia 7.0, 01/2008:1220 used to quantify the total content in % in weight of proanthocyanidins in botanical extracts comprises the following steps: reduce the fruit or plant to a powder; add ethanol (70 per cent V/V) to the powdered fruit or plant; heat under a reflux condenser and filter; wash the residue with ethanol (70 per cent V/V); add hydrochloric acid and water to the filtrate; heat under a reflux condenser; allow to cool, filter and wash the residue with ethanol (70 per cent V/V) until the filtrate is colourless; dilute the filtrate with ethanol (70 per cent V/V); evaporate this solution in a round-bottomed flask and transfer to a separating funnel; rinse the round-bottomed flask sequentially with water and transfer to the separating funnel; shake the combined solution with butanol; combine the organic layers and dilute with butanol; measure the absorbance of the solution; and calculate the percentage content of proanthocyanidins.

The HPLC method used to distinguish and measure A- and B- linkages comprises the following steps: i) a thiolytic degradation and ii) a Reversed-Phase HPLC-MS/MS Analysis.

The thiolytic degradation (i) comprises the reaction of the proanthocyanidin extract with benzyl mercaptan.

The Reversed-Phase HPLC-MS/MS analysis (ii) comprises separation of procyanidin thiolysis media on a C18 column using a solvent system consisted of A (2% acetic acid in water, v/v) and B (methanol) and identify the components in the thiolytic media.

The proportions of constituent flavan-3-ol benzylthioethers as well as proanthocyanidin A2 benzylthioether, are calculated based on the assumption that flavan-3-ol benzylthioethers derivatives and proanthocyanidin A2 benzylthioethers dimmer derivatives have the same molar absorptivities as their respective flavan-3-ol monomers and proanthocyanidin A2 dimmer.

Both, the method disclosed in the European Pharmacopeia 7.0, 01/2008:1220 and the HPLC-based method reported in Gu, L. et al (2002 and 2003), are used and described in detail in Example 4 hereinafter.

The present invention further provides a pharmaceutical composition comprising at least an A-type proanthocyanidin together with a pharmaceutically acceptable excipient, for administration to a patient for use in treating and/or preventing a mineralocorticoid receptor related disease or disorder.

The composition can be used with at least another drug to provide a combination therapy. This other drug or drugs may be part of the same composition, or may be provided as a separate composition and can be administered at the same time or at different times.

In a preferred embodiment, the A-type proanthocyanidin is used in combination with another antagonist of the mineralocorticoid receptor, preferably spironolactone or epleronone.

The term "excipient" refers to components of a drug compound other than the active ingredient (definition obtained from the European Medicines Agency- EMA). They preferably include a pharmaceutically acceptable "carrier, adjuvant and/or vehicle". Carriers are forms to which substances are incorporated to improve the delivery and the effectiveness of drugs. Drug carriers are used in drug-delivery systems such as the controlled-release technology to prolong in vivo drug actions, decrease drug metabolism, and reduce drug toxicity. Carriers are also used in designs to increase the effectiveness of drug delivery to the target sites of pharmacological actions (U.S. National Library of Medicine. National Institutes of Health). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, © 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

The pharmaceutical compositions in accordance with the invention can be adapted in order to be administered by any route of administration, be it orally or parenterally, such as pulmonarily, nasally, rectally and/or intravenously. Therefore, the formulation in accordance with the invention may be adapted for topical or systemic application, particularly for dermal, subcutaneous, intramuscular, intra-articular, intraperitoneal, pulmonary, buccal, sublingual, nasal, percutaneous, vaginal, oral or parenteral application. The preferred form of rectal application is by means of suppositories.

Suitable preparations for oral applications are tablets, pills, chewing gums, capsules, granules, drops or syrups. Suitable preparations for parenteral applications are solutions, suspensions, reconstitutable dry preparations or sprays.

The pharmaceutical composition of the invention may be formulated as deposits in dissolved form or in patches, for percutaneous application. Skin applications include ointments, gels, creams, lotions, suspensions or emulsions.

Preferably, the pharmaceutical composition of the invention is formulated for oral administration.

Another aspect of the invention is a method of treatment of a patient suffering, or likely to suffer, a mineralocorticoid receptor related disease or disorder, which comprises administering to the patient in need of such a treatment or prophylaxis a therapeutically effective amount of an A-type proanthocyanidin.

Generally an effective administered amount of a compound used in the invention will depend on the relative efficacy of the compound chosen, the severity of the disorder being treated, or the age, weight or mode of administration. However, active compounds will typically be administered once or more times a day, for example 1, 2, 3 or 4 times daily, with typical total daily doses in the range of from 0.1 to 500 mg/kg/day.

Having described the present invention in general terms, it will be more easily understood by reference to the following examples which are presented as an illustration and are not intended to limit the present invention.

### Examples

### Example 1: A-type proanthocyanidins effect on mineralocorticoid receptor expression.

A transactivation assay was carried out to measure A-type proanthocyanidins effect on mineralocorticoid receptor expression. To this end, CV-1 cells were transfected with a human mineralocorticoid receptor plasmid (in order to ever-express mineralocorticoid receptor). Following, cells were incubated for 24 hours with the next treatments:
- Control: cell medium (V)
- Aldosterone 1nM (A1nM)
- Spironolactone 100nM (S100nM)
- Aldosterone1nM+Spironolactone 100nM (A1nM+S100nM)
- A-type proanthocyanidins A 10nM, 100nM y 1µM (P10nM, 100nM and 1µM)
- Aldosterone1nM+ A-type proanthocyanidins A 10nM, 100nM and 1µM
- B-type proanthocyanidins 10nM, 100nM y 1µM (P10nM, 100nM and 1µM)
- Aldosterone1nM+ B-type proanthocyanidins 10nM, 100nM and 1µM
- Catechins 10nM, 100nM and 1µM (P10nM, 100nM and 1µM)
- Aldosterone1nM+catechins 10nM, 100nM and 1µM

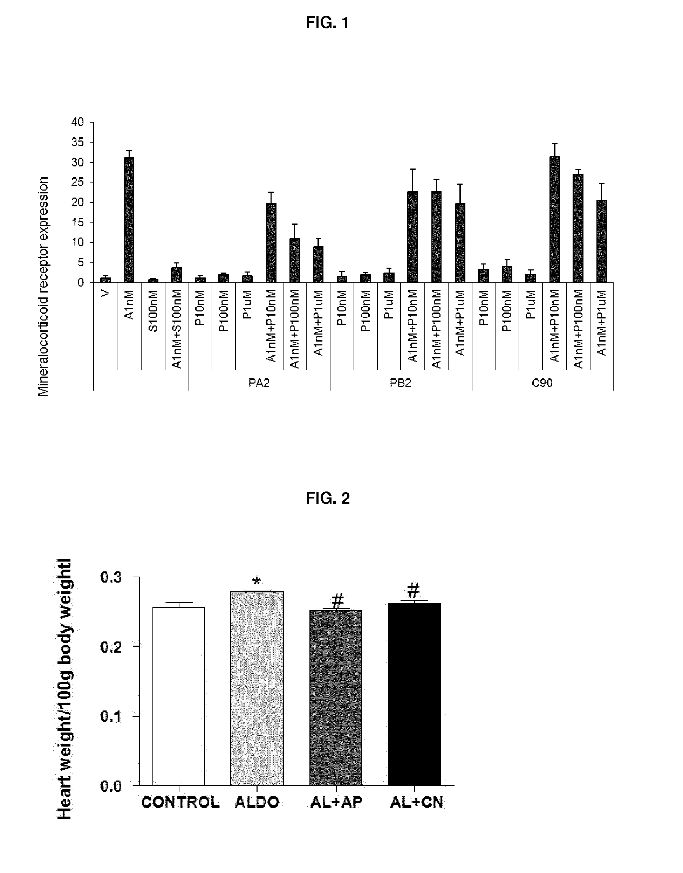

A-type proanthocyanidin effect on mineralocorticoid receptor expression was measured with a luciferase-renilla commercial kit which activity is related to steroids presence. The results obtained are shown in Figure 1. The first bar shows control and the second one shows the effect of aldosterone mineralocorticoid receptor expression which is much higher than control data. Spironolactone by itself presented a slight effect (third bar), and when together with aldosterone the bar decreases because spironolactone is blocking mineralocorticoid receptor (second and fourth bar).

Regarding to A-type proanthocyanidins, three increasing doses were tested. A-type proanthocyanidins did not show any effect on mineralocorticoid receptor expression (compare with second bar) and when are added together with aldosterone, mineralocorticoid receptor expression decreased significantly (compare with second bar). The decrease of the mineralocorticoid receptor expression is undoubtedly due to the blocking capacity of A-type proanthocyanidins, besides showing high receptor specificity as shows the dose-dependent effect. In the particular case of the highest dose, the final result is very similar to the spironolactone effect.

B-type proanthocyanidins were tested in the same concentrations as A-type and did not show any effect on the mineralocorticoid receptor when tested alone (compare with second bar). When incubated together with aldosterone, did not decrease mineralocorticoid receptor expression.

Catechins did not show any effect the mineralocorticoid receptor when tested alone or together with aldosterone (compare with second bar).

### Example 2: effect of A-type proanthocyanidins-rich natural extracts and B-type proanthocyanidins-rich natural extracts in cardiac alterations in aldosterone-treated rats.

The study was carried out to compare the effects of two proanthocyanidins-rich natural extracts: apple ("B-type proanthocyanidins-rich" wherein there are not A-type proanthocyanidins) and cinnamon (A-type proanthocyanidins-rich: 6.5 % A-type linkages) in cardiac alterations in aldosterone-treated rats.

Male Wistar rats (300g) were used in the study according to the guidelines for ethical care of experimental animals of the European Union and granted by the Universidad Complutense Ethics Review Board. Rats were fed standard rat chow and tap water ad libitum and kept in a quiet room at constant temperature (20 to 22°C) and humidity (50 to 60%). A group of animals received aldosterone (ALDO 1000µg/kg/day, subcutaneously) and 1% NaCl in drinking water during 21 days; a second group of rats received aldosterone (ALDO 1000µg/kg/day) and 1% NaCl and was treated with cinnamon extract (AL+CN 50mg/kg/day) during 21 days; a third group which received aldosterone (ALDO 1000µg/kg/day) and 1 % NaCl in drinking water and was treated with apple extract (AL+AP 50mg/kg/day) in the drinking water ; and as a control group rats which received the vehicle 0.1 mL corn oil/rat/day during 21 days.

At the end of the treatment period, animals were intraperitoneal anesthetized (ketamine Imalgene 1000, 70mg/Kg, and xylazine Rompun 2%, 6mg/Kg) and a catheter (Science FT211 B of 1.6F diameter; Ontario, Canada) was inserted into the right carotid. Then, the catheter was advanced to the left ventricle and left ventricular end diastolic pressure (LVEDP). The catheter was connected to a data acquisition system. Signals were monitored and digitally stored for analysis and after measuring hemodynamic parameters, the animals were sacrificed by exsanguination; the heart was removed, weighed and rapidly frozen in liquid nitrogen for molecular studies. The ratio of the heart to body weight was used as the index of cardiac hypertrophy.

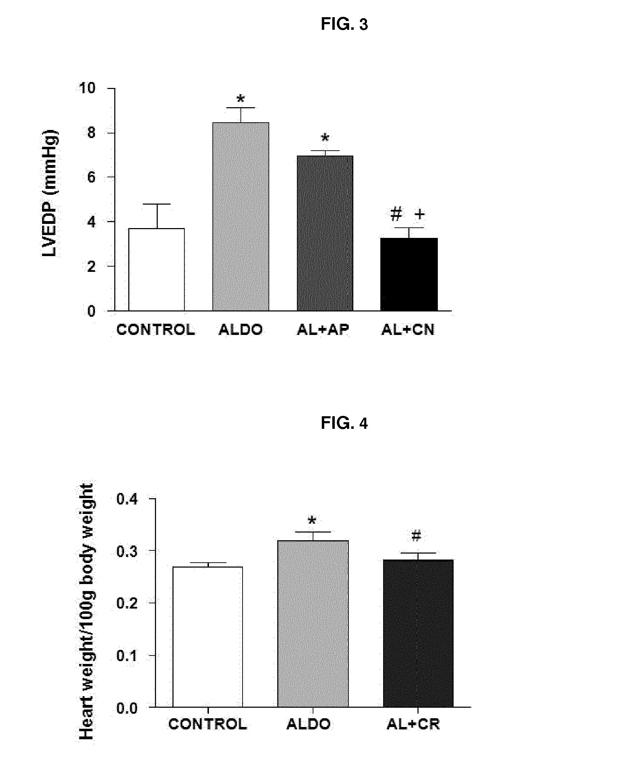

Cinnamon extract, A-type proanthocyanidins-rich, significantly improved the diastolic dysfunction induced by aldosterone reducing LVEDP to control levels, therefore, improving cardiac function of the animals (table 1 and figure 2). On the contrary, apple extract, B-type proanthocyanidins-rich, was unable to reduce the diastolic dysfunction induced by aldosterone keeping significant high levels compared to the control group (table 1 and figure 2). Cardiac hypertrophy is similar when compared both extracts. Cardiac hypertrophy is a compensatory mechanism difficult to reverse (table 1 and figure 3). The remarkable aspect is that when treating with cinnamon extract (A-type proanthocyanidins-rich) the animals were able to compensate the cardiac hypertrophy and to maintain a normalized cardiac function. This does not occur when treating with apple extract (B-type proanthocyanidins-rich).

**Table 1. LVEDP: left ventricle end diastolic pressure and HRW: heart relative weight. *p<0,05 vs CONTROL, #p<0,05 vs ALDO, +p<0,05 vs ALDO+APPLE.**

| | CONTROL | ALDO | ALDO+APPLE | ALDO+CINNAMON |
|---|---|---|---|---|
| LVEDP (mmHg) | 3,70±1,1 | 8,46±0,6* | 6,98±0,2* | 3,28±0,5^{#+} |
| HRW (mg/g) | 0,256±0,007 | 0,278±0,001* | 0,252±0,004^{#} | 0,262±0,001^{#} |

### Example 3: effect of highly A-type proanthocyanidins-rich natural extract in cardiac alterations in aldosterone-treated rats.

The study was carried out to analyze the effects of a cranberry extract which is a highly A-type proanthocyanidins-rich natural extract (66.0% A-type linkages) in cardiac alterations in aldosterone-treated rats. To this end a low dose was used to show both effect and efficacy of A-type proanthocyanidins.

Male Wistar rats (300g; Harlan Ibérica, Barcelona, Spain) were used in the study according to the guidelines for ethical care of experimental animals of the European Union and granted by the Universidad Complutense Ethics Review Board. Rats were fed standard rat chow and tap water ad libitum and kept in a quiet room at constant temperature (20 to 22°C) and humidity (50 to 60%). A group of animals received aldosterone (ALDO 1000µg/kg/day, subcutaneously) and 1% NaCl in drinking water during 21 days; a second group of rats received aldosterone (ALDO 1000µg/kg/day) and 1% NaCl and was treated with cranberry extract (AL+CR 5mg/kg/day) during 21 days; and as a control group rats which received the vehicle 0.1mL corn oil/rat/day during 21 days.

At the end of the treatment we used the same methodology as in example 2.

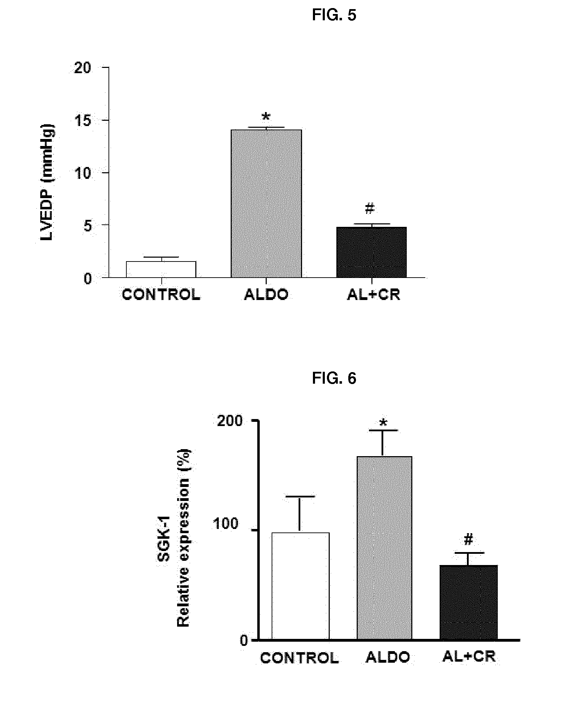

Cranberry extract reduced to control levels cardiac hypertrophy as well as LVEDP when compare to aldosterone group (table 2, figure 4 and 5).

We also measured genic expression of SGK-1. As figure 6 shows, genic levels of SGK-1 in aldosterone group were significantly higher when compared with control group. The treatment with cranberry extract reduced the levels of this main aldosterone-actions mediator to control levels showing one more time the relation between A-type proanthocyanidins and mineralocorticoid receptor.

**Table 2. LVEDP: left ventricle end diastolic pressure and HRW: heart relative weight. *p<0,05 vs CONTROL, #p<0,05 vs ALDO.**

| | CONTROL | ALDO | ALDO+CRANBERRY |
|---|---|---|---|
| LVEDP (mmHg) | 1.52±0.45 | 14.10±0.20* | 4.85±0.21^{#} |
| HRW (mg/g) | 0.263±0.009 | 0.320±0.016* | 0.283±0.013^{#} |

### Example 4. Total proanthocyanidins content (European pharmacopea and DMAC methods)

Three different analytical procedures were employed to quantify proanthocyanidins in three botanical extracts, according to three different methodologies. Two of these methods:
- DMAC (Prior, RL. et al (2010), "Multi-laboratory validation of a standard method for quantifying proanthocyanidins in cranberry powders", Journal of the Science of Food and Agriculture, 90 (9), 1473-1478), and
- European Pharmacopeia (*European Pharmacopeia 7.0,* 01/2008:1220)*,*
are widely used to quantify the total content of proanthocyanidins in botanical extracts. However, both methods cannot differentiate between A-type and B-type proanthocyanidins.

The third method, based on HPLC technique, is able to distinguish A- and B-linkages (Gu, L. et al (2003) "Screening of Foods Containing Proanthocyanidins and Their Structural Characterization Using LC-MS/MS and ThiolyticDegradation", Journal of Agricultural and Food Chemistry, 51 (25), 7513-7521; and Gu, L. et al (2002) "Fractionation of Polymeric Procyanidins from Lowbush Blueberry and Quantification of Procyanidins in Selected Foods with an Optimized Normal-Phase HPLC-MS Fluorescent Detection Method", Journal of Agricultural and Food Chemistry, 50 (17), 4852-4860). A thiolytic degradation was conducted which allowed to specifically hydrolyze the B-type bond in its monomers, yielding the A-type polymers untouched. This methodology permitted to determinate the proportion of A-type linkages in the extracts studied.

| **European Pharmacopeia.** | | | |
|---|---|---|---|
| **Herbal extracts** | **Botanical origin** | **% PACs (% w/w)** | |
| **Cinnamon, dry extract** | *Cinnamomum zeylanicum* | 34,3 | |
| **Almond skin, dry extract** | *Prunus dulcis* | 25,1 | |
| **Cranberry, dry extract** | *Vaccinium macrocarpon* | 86,5 | |

| **DMAC Method.** | | | |
|---|---|---|---|
| **Herbal extracts** | **Botanical origin** | | **µmol Proantho. B1 Eq / g** |
| **Cinnamon, dry extract** | *Cinnamomum zeylanicum* | | 112,4 |
| **Almond skin, dry extract** | *Prunus dulcis* | | 122,7 |
| **Cranberry, dry extract** | *Vaccinium macrocarpon* | | 274,8 |

| **HPLC: relative proportion between A-type and B-type proanthocyanidins** | | | |
|---|---|---|---|
| **Herbal extracts** | **Botanical origin** | **% A-Type Linkages** | **% B-Type Linkages** |
| **Cinnamon, dry extract** | *Cinnamomum zeylanicum* | 6,5 | 93,5 |
| **Almond skin, dry extract** | *Prunus dulcis* | 2,9 | 97,1 |
| **Cranberry, dry extract** | *Vaccinium macrocarpon* | 66,0 | 34,0 |

The method described in the European Pharmacopeia 7.0, 01/2008:1220 used to quantify the total content of proanthocyanidins in botanical extracts was performed by following steps: reduce the fruit or plant to a powder; add 30 mL of ethanol (70 per cent V/V) to 2.50 g of the powdered fruit or plant; heat under a reflux condenser for 30 min and filter; wash the residue with 10.0 mL of ethanol (70 per cent V/V); add to the filtrate 15.0 mL of hydrochloric acid and 10.0 mL of water; heat under a reflux condenser for 80 min; allow to cool, filter and wash the residue with ethanol (70 per cent V/V) until the filtrate is colourless; dilute the filtrate to 250.0 mL with ethanol (70 per cent V/V); evaporate 50.0 mL of this solution in a round-bottomed flask to about 3 mL and transfer to a separating funnel; rinse the round-bottomed flask sequentially with 10 mL and 5 mL of water and transfer to the separating funnel; shake the combined solution with 3 quantities, each of 15 mL, of butanol; combine the organic layers and dilute to 100.0 mL with butanol; measure the absorbance of the solution at 545 nm; and calculate the percentage content of proanthocyanidins, expressed as cyanidin chloride (i.e. taking the specific absorbance of cyanidin chloride to be 75) using the following expression: %=(Ax500)/(75xm)
A = absorbance at 545 nm,
m = mass of the substance to be examined, in grams.

The HPLC method used to distinguish and measure A- and B- linkages described in Gu, L. et al Journal of Agricultural and Food Chemistry, 2003, 51 (25), 7513-7521; and Gu, L. et al, Journal of Agricultural and Food Chemistry, 2002, 50 (17), 4852-4860) comprises the following steps:
i) Thiolytic Degradation:
   The proanthocyanidin extract was dissolved in methanol at 5 mg/ml of proanthocyanidins, 150 µL was put into a 250- µL polypropylene insert. Then, it was mixed with 50 µL of methanol acidified with concentrated HCl (3.3%, v/v) and 100 µL of benzyl mercaptan (5% v/v in methanol). The inserts were put into a 1.5-mL vial and sealed with an inert Teflon cap. The reaction was carried out at 50 ºC for 25 min. At the end of the reaction, the reaction mixtures were kept in the freezer (-18 °C) before 10 µL was injected for reversed phase HPLC-MS/MS analysis.
ii) Reversed-Phase HPLC-MS/MS Analyses:
   Separation of procyanidin thiolysis media was performed on an Agilent-Technologies system. A 250 x 4.6 mm i.d., 5 µm, C18 column was used at 25 °C. For separation, the solvent system consisted of A (2% acetic acid in water, v/v) and B (methanol) at a flow rate of 1 ml/min. The gradient was 0-55 min, 10-63% B, 55-57 min, 63-100% B, then isocratic at 100% B during 10 min and re-equilibration for 20 min.

The detection wavelength of the diode array detector was set at 280 nm for catechin, epicatechin, proanthocyanidins and their benzylthioethers. Components in the thiolytic media were identified by comparing their retention times with primary reference standards of catechin, epicatechin and proanthocyanidin A2. Concerning the benzilthioether derivatives, they were identified with a mass spectrometer equipped with electrospray ionization (ESI) and operated in the negative ion mode, at a voltage of 3 kV.

The terminal units of the proanthocyanidins are released as free flavan-3-ols after the thiolytic degradation, which cannot be distinguished from the flavan-3-ol monomers in the sample by HPLC.

The proportions of constituent flavan-3-ol benzylthioethers as well as proanthocyanidin A2 benzylthioether, were calculated based on the assumption that flavan-3-ol benzylthioethers derivatives and proanthocyanidin A2 benzylthioethers dimmer derivatives, have the same molar absorptivities as their respective flavan-3-ol monomers and proanthocyanidin A2 dimmer. They were quantified by comparison with authentic standards, +(-)catechin, -(-)epicatechin and proanthocyanidin A2, (Extrashyntese, Genay, France).

## Claims

1. A-type proanthocyanidin for use in treating and/or preventing a mineralocorticoid receptor related disease or disorder.

2. A-type proanthocyanidin for use according to claim 1 wherein the mineralocorticoid receptor related disease or disorder is selected from the group consisting of cardiac hypertrophy, cardiac fibrosis, left ventricle diastolic dysfunction, heart failure, heart attack and acute coronary syndromes.

3. A-type proanthocyanidin for use according to claim 1 wherein the mineralocorticoid receptor related disease or disorder is hypertension.

4. A-type proanthocyanidin for use according to claim 1 wherein the mineralocorticoid receptor related disease or disorder is selected from the group consisting of arteriosclerosis, aterotrombosis and ateromatosis.

5. A-type proanthocyanidin for use according to claim 1 wherein the mineralocorticoid receptor related disease or disorder is selected from the group consisting of chronic kidney failure, acute kidney failure, nephritic syndrome and its hepatosplacnic and gastro-intestinal alterations.

6. A-type proanthocyanidin for use according to claim 1 wherein the mineralocorticoid receptor related disease or disorder is selected from the group consisting of hepatosplacnic and gastro-intestinal alterations.

7. A-type proanthocyanidin for use according to claim 1 wherein the mineralocorticoid receptor related disease or disorder is related to steroids hormones selected from glucocorticoids and mineralocorticoids.

8. A-type proanthocyanidin for use according to claim 7 wherein the disease or disorder related to steroids hormones is selected from the group consisting of primary and secondary hyperaldosteronism, non-aldosterone mediated hypermineralocorticoidism, hypercortisolsm and hyperglucocorticoism.

9. A-type proanthocyanidin for use according to any one of preceding claims wherein the A-type proanthocyanidin is present in a natural extract comprising at least 5% in weight of proanthocyanidins, wherein at least 2% of the linkages of said proanthocyanidins are A-type linkages.

10. A-type proanthocyanidin for use according to any one of preceding claims wherein the A-type proanthocyanidin is present in a natural extract selected from the group consisting of horse-chestnut extract, cinnamon extract, almond extract, avocado extract, peanut extract and cranberry extract.

11. Pharmaceutical composition comprising at least one A-type proanthocyanidin and at least one pharmaceutically acceptable excipient for use in treating and/or preventing a mineralocorticoid receptor related disease or disorder.

12. Pharmaceutical composition according to claim 11 further comprising at least another drug to provide a combination therapy.
